(19) Europäisches Patentamt
European Patent Office
Office européen des brevets

(11) Veröffentlichungsnummer: **0 216 228**
**A1**

(12) # EUROPÄISCHE PATENTANMELDUNG

(21) Anmeldenummer: 86112300.8

(22) Anmeldetag: 05.09.86

(51) Int. Cl.[4]: C 07 D 305/06
C 07 D 331/04, C 07 D 407/04
C 07 D 409/04, C 07 D 409/08
C 07 D 409/12, A 01 N 43/20

(30) Priorität: 09.09.85 DE 3532478

(43) Veröffentlichungstag der Anmeldung: 01.04.87 Patentblatt 87/14

(84) Benannte Vertragsstaaten: AT BE CH DE FR GB IT LI LU NL SE

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Franke, Heinrich, Dr.**
**Fabeckstrasse 38**
**D-1000 Berlin 37(DE)**

(72) Erfinder: **Joppien, Hartmut, Dr.**
**Juttastrasse 18**
**D-1000 Berlin 37(DE)**

(72) Erfinder: **Franke, Helga, Dr.**
**Karl-Stieler-Strasse 2b**
**D-1000 Berlin 41(DE)**

(54) **Oxetan- und Thietanderivate mit pestizider Wirkung.**

(57) There are described new compounds of general formula I

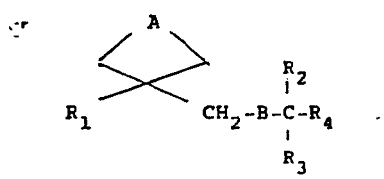

(I)

in which

$R_1$ is aryl or aryl substituted by $C_{1-4}$-alkyl, halo-$C_{1-4}$-alkyl, phenyl-$C_{1-4}$-alkyl, $C_{2-4}$-alkenyl, halo-$C_{2-4}$-alkenyl, phenyl-$C_{2-4}$-alkenyl, $C_{2-4}$-alkynyl, halo-$C_{2-4}$-alkynyl, phenyl-$C_{2-4}$-alkynyl, $C_{1-4}$-alkoxy, halo-$C_{1-4}$-alkoxy, phenyl-$C_{1-4}$-alkoxy, $C_{2-4}$-alkenyloxy, alkylsulphonyloxy, haloalkylsulphonyloxy, arylsulphonyloxy, halo-$C_{2-4}$-alkenyloxy, phenyl-$C_{2-4}$-alkenyloxy, halo, cyano, nitro, aryloxy, haloaryloxy, $C_{1-4}$-alkylaryloxy, or nitro-$C_{1-4}$-alkylaryloxy,

$R_2$ and $R_3$ are the same or different and are hydrogen, fluorine, cyano or ethynyl,

$R_4$ is phenyl or pyridyl or these groups substituted by one or more of $C_{1-6}$-alkyl, halo-$C_{1-6}$-alkyl, phenyl-$C_{1-6}$-alkyl, $C_{1-6}$-alkyl interrupted by an O–, N– or S– atom, $C_{2-4}$-alkenyl, halo-$C_{2-4}$-alkenyl, phenyl-$C_{2-4}$-alkenyl, $C_{1-4}$-alkoxy, halo-$C_{1-4}$-alkoxy, phenyl-$C_{1-4}$-alkoxy, $C_{2-4}$-alkenyloxy, halo-$C_{2-4}$-alkenyloxy, phenyl-$C_{2-4}$-alkenyloxy, aryloxy, haloaryloxy, $C_{1-4}$-alkylaryloxy, arylamino, haloarylamino, $C_{1-4}$-alkylarylamino, aryl–*N*–$C_{1-4}$-alkylamino, aryl–*N*–$C_{1-4}$acylamino, aroyl, haloaroyl, $C_{1-4}$-alkylaroyl, aryl, haloaryl, $C_{1-4}$-alkylaryl or halo,

A is O or S and

B is $CH_2$ or O,

processes for their preparation and their use as insecticides and acaricides.

EP 0 216 228 A1

This invention relates to oxetane and thietane derivatives and their preparation, as well as pesticidal compositions based on these compounds.

Synthetic pyrethroids are a well known group of inseticides. In recent years there have been described pyrethroids in which the conventional ester grouping has been replaced by an ether or alkylene chain as for example in EP 94,085, EP 104,908 and DE 3,317,908. We are not aware however of compounds of this type containing an oxetane or thietane group in the main chain.

It has now been found that compounds according to the invention possess particularly valuable insecticidal and acaricidal properties. The compounds are of general formula I

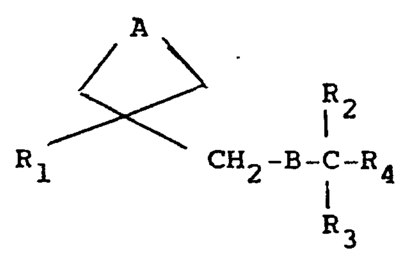

(I)

in which

$R_1$ is aryl or aryl substituted by $C_{1-4}$-alkyl, halo-$C_{1-4}$-alkyl, phenyl-$C_{1-4}$-alkyl, $C_{2-4}$-alkenyl, halo-$C_{2-4}$-alkenyl, phenyl-$C_{2-4}$-alkenyl, $C_{2-4}$-alkynyl, halo-$C_{2-4}$-alkynyl, phenyl-$C_{2-4}$-alkynyl, $C_{1-4}$-alkoxy, halo-$C_{1-4}$-alkoxy,

phenyl-$C_{1-4}$-alkoxy, $C_{2-4}$-alkenyloxy, alkylsulphonyloxy, haloalkylsulphonyloxy, arylsulphonyloxy, halo-$C_{2-4}$-alkenyloxy, phenyl-$C_{2-4}$-alkenyloxy, halo, cyano, nitro, aryloxy, haloaryloxy, $C_{1-4}$-alkylaryloxy, or nitro-$C_{1-4}$-alkylaryloxy,

$R_2$ and $R_3$ are the same or different and are hydrogen, fluorine, cyano or ethynyl,

$R_4$ is phenyl or pyridyl or these groups substituted by one or more of $C_{1-6}$-alkyl, halo-$C_{1-6}$-alkyl, phenyl-$C_{1-6}$-alkyl, $C_{1-6}$-alkyl interrupted by an O-, N- or S- atom, $C_{2-4}$-alkenyl, halo-$C_{2-4}$-alkenyl, phenyl-$C_{2-4}$-alkenyl, $C_{1-4}$-alkoxy, halo-$C_{1-4}$-alkoxy, phenyl-$C_{1-4}$-alkoxy, $C_{2-4}$-alkenyloxy, halo-$C_{2-4}$-alkenyloxy, phenyl-$C_{2-4}$-alkenyloxy, aryloxy, haloaryloxy, $C_{1-4}$-alkylaryloxy, arylamino, haloarylamino, $C_{1-4}$-alkylarylamino, aryl-N-$C_{1-4}$-alkylamino, aryl-N-$C_{1-4}$ acylamino, aroyl, haloaroyl, $C_{1-4}$-alkylaroyl, aryl, haloaryl, $C_{1-4}$-alkylaryl or halo,

A is O or S and

B is $CH_2$ or O.

The aryl group designated as $R_1$ in general formula I also includes 1-naphthyl, 2-naphthyl, benzofuran-5-yl,

benzothiophen-5-yl, benzofuran-6-yl, benzothiophen-6-yl, benzoxazol-5-yl, benzoxazol-6-yl, indan-5-yl, indan-6-yl, 1,4-benzodioxan-6-yl, 1,3-benzodioxan-6-yl, 1,3-benzodioxan-7-yl and 1,3-benzodioxol-5-yl.

A particularly preferred group of compounds is that in which $R_2$ and $R_3$ are both hydrogen and $R_4$ is 3-phenoxyphenyl or 4-fluoro-3-phenoxyphenyl. A is preferably O and B is preferably $CH_2$. $R_1$ is preferably phenyl, optionally substituted in the 4-position or in both the 3- and 4- positions by halogen, $C_{1-4}$-alkyl, $C_{1-4}$-alkoxy, $C_{1-4}$-haloalkyl or $C_{1-4}$-haloalkoxy or two adjacent substituents form a methylenedioxy group. Particularly preferred substituents are halogen (especially chlorine and fluorine), methyl, trifluoromethyl and ethoxy.

The oxetanes of the invention of formula I in which A is oxygen can be prepared by treating a compound of formula II

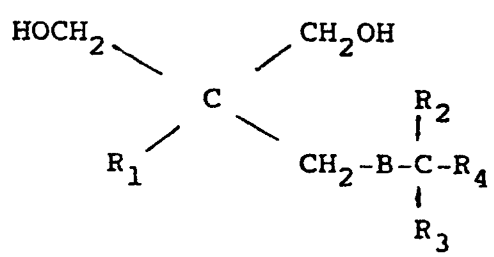

(II)

firstly with a sulphonyl chloride to give a compound of formula III

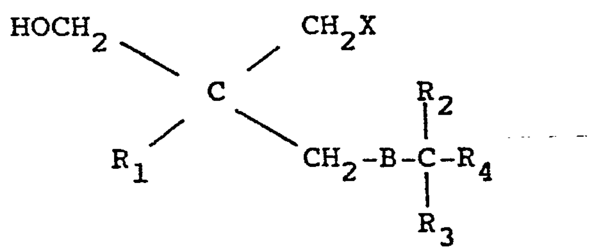
(III)

in which $R_1$, $R_2$, $R_3$, $R_4$ and B have the meanings given above and X is a leaving group, such as halogen, mesylate or tosylate, and then cyclising this to the oxetane, with a strong base.

The cyclisation reaction is generally carried out in solution. Suitable bases are metal alcoholates, such as for example potassium tert.-butylate, metal hydrides, such as for example sodium hydride, metal amides, such as for example lithium diisopropylamide and metal alkyl compounds, such as for example ethyl magnesium bromide or butyl lithium.

Suitable solvents, as opposed to the reactants, especially the bases, include inert substances such as aliphatic and aromatic hydrocarbons such as for example hexane, benzene or toluene and ethers such as for example diethyl ether, tetrahydrofuran or dimethoxyethane. Further, amides, such as for example dimethylformamide and diethylformamide may be suitable.

The reaction is carried out at a temperature of -78 to 140°C, preferably at 20-80°C and usually at atmospheric pressure.

It is particularly advantageous to carry out both reaction stages in a so-called one-pot reaction as is described in "P. Picard, D. Leclercq, J.-P. Bats, J. Moulines, Synthesis 550 (1981)".

The thietanes of the invention of formula I in which A is sulphur can be prepared by treating a compound of formula II with a sulphonyl chloride to give an intermediate of general formula IV

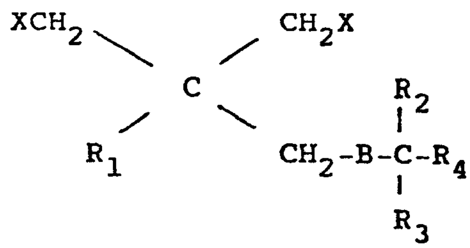

(IV)

in which $R_1$, $R_2$, $R_3$, $R_4$, X and B have the meanings given above and then cyclising this to the thietane with sodium sulphide.

The cyclisation reaction is generally carried out in solution. It is especially advantageous to use dimethyl sulphoxide as solvent and a reaction temperature of 80-100°C.

The diols used as starting materials can be prepared in a two stage synthesis in which a substituted malonate ester of the general formula

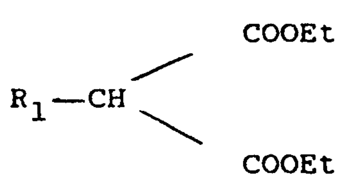

is reacted with an alkylating agent of general formula

$$X-CH_2-B-\overset{\displaystyle R_2}{\underset{\displaystyle R_3}{\overset{|}{\underset{|}{C}}}}-R_4$$

in which $R_1$, $R_2$, $R_3$, $R_4$, X and B have the meanings given above, in the presence of a base and then the disubstituted malonate ester is reduced to the diol with lithium aluminium hydride.

The compounds of the invention obtained by these processes can be isolated in conventional manner for example by distillation of the solvent at normal or reduced pressures, by precipitation with water or by extraction.

A high degree of purity can be achieved as a general rule by column chromatography as well as by fractional distillation or crystallisation.

The compounds are colourless and odourless oils that dissolve well in practically all organic solvents but are almost insoluble in water.

The compounds of the invention have insecticidal and acaricidal activity and are particularly useful in combating a variety of economically important insects, and acarids including animal ectoparasites. Examples include Lepidoptera, such as <u>Plutella xylostella</u>, <u>Spodoptera littoralis</u>, <u>Heliothis armigera</u>, and <u>Pieris brassicae</u>;

Diptera, such as *Musca domestica*, *Ceratitis capitata*, *Erioischia brassicae*, *Lucilia sericata* and *Aedes aegypti*; Homoptera, including aphids such as *Megoura viciae* and *Nilaparvata lugens*; Coleoptera, such as *Phaedon cochleariae*, *Anthonomus grandis* and *Epilachna varivestis* and corn rootworms (*Diabrotica* spp., e.g. *Diabrotica undecimpunctata*); Orthoptera, such as cockroaches e.g. *Blattella germanica*; Hymenoptera, such as ants e.g. *Monomorium pharaonis*; mange mites, e.g. *Sarcoptes* spp.; ticks, such as *Boophilus microplus* and lice, such as *Damalinia bovis* and *Linognathus vituli*; as well as spider mites such as *Tetranychus urticae* and *Panonychus ulmi*.

The compounds are distinguished by a surprisingly high level of activity against important pest species, especially ticks, which is greater than that of known pesticidal compositions having a similar mode of action.

The compounds according to the invention can be used at a concentration of 0.0005 to 5%, preferably from 0.001 to 1%, calculated as gram active material per 100ml of the composition.

The compounds of the invention can be used either alone or in mixture with each other or another insecticide. Optionally other plant protection or pesticidal compositions, such as for example insecticides, acaricides or fungicides can be added depending on the

desired result.

An improvement in the intensity and speed of action can be obtained, for example, by addition of suitable adjuvants, such as organic solvents, wetting agents and oils. Such additives may allow a decrease in the dose.

Suitable mixture partners may include phospholipids, e.g. phosphatidylcholine, hydrated phosphatidylcholines, phosphatidylethanolamine, N-acyl-phosphatidylethanol-amines, phosphatidylinositol, phosphatidylserine, lysolecithin or phosphatidylglycerol.

The designated active ingredients or their mixtures can suitably be used, for example, as powders, dusts, granules, solutions, emulsions or suspensions, with the addition of liquid and/or solid carriers and/or diluents and, optionally, binding, wetting, emulsifying and/or dispersing adjuvants.

Suitable liquid carriers are, for example, water, aliphatic and aromatic hydrocarbons such as benzene, toluene, xylene, cyclohexanone, isophorone, dimethyl sulphoxide, dimethylformamide, other mineral-oil fractions and plant oils.

Suitable solid carriers include mineral earths, e.g. bentonite, silica gel, talcum, kaolin, attapulgite, limestone, silicic acid and plant products, e.g. flours.

As surface-active agents there can be used for example

calcium lignosulphonate, polyoxyethylenealkylphenyl ether, naphthalenesulphonic acids and their salts, phenolsulphonic acids and their salts, formaldehyde condensates, fatty alcohol sulphates, as well as substituted benzenesulphonic acids and their salts.

The percentage of the active ingredient(s) in the various preparations can vary within wide limits. For example, the compositions can contain about 10 to 90 percent by weight active ingredients, and about 90 to 10 percent by weight liquid or solid carriers, as well as, optionally up to 20 percent by weight of surfactant.

The agents can be applied in customary fashion, for example with water as the carrier in spray mixture volumes of approximately 100 to 3,000 l/ha. The agents can be applied using low-volume or ultra-low-volume techniques or in the form of so-called microgranules.

The preparation of these formulations can be carried out in a known manner, for example by milling or mixing processes. Optionally, individual components can be mixed just before use for example by the so-called commonly used tank-mixing method.

Formulations can be prepared, for example, from the following ingredients.

a) 80 percent by weight active ingredient
15 percent by weight kaolin
5 percent by weight surface-active agent based on the sodium salt of N-methyl-N-oleyltaurine and the calcium lignosulphonate

b) 45 percent by weight active ingredient
5 percent by weight sodium aluminium silicate
15 percent by weight cetylpolyglycol ether with 8 moles ethylene oxide
2 percent by weight spindle oil
10 percent by weight polyethylene glycol
23 parts water

c) 20 percent by weight active ingredient
35 percent by weight bentonite
8 percent by weight calcium lignosulphonate
2 percent by weight of the sodium salt of N-methyl-N-oleyltaurine
35 percent by weight silicic acid

d) 20 percent by weight active ingredient
75 percent by weight isophorone
5 percent by weight of an emulsifier mixture of calcium phenylsulphonate and fatty alcohol polyglycol ether

The following examples illustrate the preparation of compounds acording to the invention.

Example 1

3-(4-Ethoxyphenyl)-3-[3-(3-phenoxyphenyl)propyl]oxetane

A solution of butyl lithium in n-hexane (4.7 ml, 1.6M) was added dropwise at 0°C to 2-(4-ethoxyphenyl)-2-[3-(3-phenoxyphenyl)propyl]propane-1,3-diol (2.8g), dissolved in tetrahydrofuran (THF)(15ml). After 10 minutes, p-toluenesulphonyl chloride (1.3g) dissolved in THF (10ml) was added dropwise at 0°C and after another 45 minutes more butyl lithium solution in n-hexane (4.7 ml, 1.6M) was added. After stirring for one hour at 0°C and 12 hours at room temperature, the mixture was added to ice-water, extracted three times with ether, the extract washed with water, dried over magnesium sulphate and evaporated. After chromatography on silica-gel using ether/hexane (1:9) as eluent, there was obtained 1.6g of product (60% of theory).

$n_D^{20}$:1.5784

Preparation of starting material

Sodium hydride (6.2 g of a 55% dispersion) was washed free of oil with toluene and then treated with dimethyl sulphoxide (300 ml), sodium iodide (5.5 g) and 1-bromo-3-(3-phenoxyphenyl)propane (36.4 g; 125 mmol). Diethyl 4-ethoxyphenylmalonate (40 g, 143 mmol) was added,

dropwise, at room temperature and then stirred for twenty hours at this temperature. The mixture was added to ice-water, extracted three times with ether, the extracts washed with water, dried over magnesium sulphate and evaporated. After chromatography on silica-gel, there was obtained 53.4 g of diethyl 2-(4-ethoxyphenyl)-2-[3-(3-phenoxyphenyl)-propyl]malonate (87% of theory). $n_D^{20}$:1.5456.

A solution of this product (25 g, 51 mmol) in ether (170 ml) was added dropwise to lithium aluminium hydride (3.9 g, 102 mmol) in ether (170 ml). The mixture was heated under reflux for one hour and then carefully hydrolysed, first with water and then 10% sulphuric acid. It was then extracted three times with ethyl acetate, the extract washed with water, dried over magnesium sulphate and evaporated to give 19.8 g of 2-(4-ethoxyphenyl)-2-[3-(3-phenoxyphenyl)propyl]propane-1,3-diol. $n_D^{20}$:1.5860

Example 2

3-(4-Ethoxyphenyl)-3-[3-(3-phenoxyphenyl)propyl]thietane

2-(4-Ethoxyphenyl)-2-[3-(3-phenoxyphenyl)propyl]propane-1,3-diol ditosylate (39.5 g) and sodium sulphide ($Na_2S.9H_2O$; 13.3 g) were stirred in dimethyl sulphoxide (300 ml) at 90°C for 3 hours. The mixture was then poured into ice-water, extracted with ether, dried

and evaporated. The crude product was purified by silica gel chromatography to give 17.0 g of product as a colourless oil (76% of theory).
$n_D^{20}$:1.5976.

Preparation of the starting material

2-(4-Ethoxyphenyl)-2-[3-(3-phenoxyphenyl)propyl]propane-1,3-diol (25 g) was dissolved in pyridine (150 ml) and 4-dimethylaminopyridine added (20 mg). p-Toluenesulphonyl chloride (23.4 g), dissolved in pyridine (20 ml), was added dropwise at -5°C. The mixture was allowed to rise to room temperature over 2 hours and stirred overnight. It was then added to a hydrochloric acid-ice mixture, extracted with ether and the extract dried and evaporated to give 40.1 g of crude product which was used without further purification.

Example 3

3-(4-Ethoxyphenyl)-3-(4-fluoro-3-phenoxybenzyloxymethyl)-oxetane

In a similar manner to Example 1, starting from 2-(4-ethoxyphenyl)-2-(4-fluoro-3-phenoxybenzyloxymethyl)-propane-1,3-diol, there was obtained the title compound as an oil, $n_D^{20}$:1.5699

Preparation of starting material

Sodium (2.18 g, 35 mmol) was added to diethyl 4-ethoxyphenylmalonate (26.6 g, 95 mmol) dissolved in

dioxane (100 ml) and refluxed until the sodium had dissolved. Chloromethyl (4-fluoro-3-phenoxybenzyl) ether (25.4 g, 95 mmol) was added, dropwise, at 40-50°C and then heated under reflux for 3 hours. The mixture was added to ice-water, extracted three times with ether, the extracts washed with water, dried over magnesium sulphate and evaporated. After chromatography on silica-gel, using toluene as the eluent, there was obtained 25.3 g of diethyl 2-(4-ethoxyphenyl)-2-(4-fluoro-3-phenoxybenzyloxymethyl)malonate (52% of theory). This was then reduced in a similar manner to Example 1 using lithium aluminium hydride to give crude 2-(4-ethoxyphenyl)-2-(4-fluoro-3-phenoxybenzyloxymethyl)propane-1,3-diol.

In a similar manner the following compounds according to the invention were prepared.

| Example No. | Compound | Physical constant $n_D^{20}$ or mp. |
|---|---|---|
| 4 | 3-[3-(3-phenoxyphenyl)propyl]-3-phenyloxetane | 1.5838 |
| 5 | 3-(4-chlorophenyl)-3-[3-(3-phenoxyphenyl)-propyl]oxetane | 1.5884 |
| 6 | 3-(3,4-dimethoxyphenyl)-3-[3-(3-phenoxyphenyl)propyl]oxetane | 1.5824 |

| Example No. | Compound | Physical constant $n_D^{20}$ or mp. |
|---|---|---|
| 7 | 3-(4-chlorophenyl)-3-(3-phenoxybenzyloxymethyl)oxetane | 1.5840 |
| 8 | 3-(4-ethoxyphenyl)-3-(3-phenoxybenzyloxymethyl)oxetane | 1.5798 |
| 9 | 3-(4-methoxyphenyl)-3-[3-(3-phenoxyphenyl)propyl]oxetane | 1.5867 |
| 10 | 3-(4-ethoxyphenyl)-3-[3-(4-fluoro-3-phenoxyphenyl)propyl]oxetane | 1.5673 |
| 11 | 3-(4-chlorophenyl)-3-[3-(4-fluoro-3-phenoxyphenyl)propyl]oxetane | 1.5760 |
| 12 | 3-(4-chlorophenyl)-3-(4-fluoro-3-phenoxybenzyloxymethyl)oxetane | 78-80°C |
| 13 | 3-(4-chlorophenyl)-3-[3-(3-phenoxyphenyl)propyl]thietane | 1.6137 |
| 14 | 3-(4-chlorophenyl)-3-(3-phenoxybenzyloxymethyl)thietane | 1.6082 |
| 15 | 3-(4-ethoxyphenyl)-3-(3-phenoxybenzyloxymethyl)thietane | 1.6000 |
| 16 | 3-[3-(3-phenoxyphenyl)propyl]-3-phenylthietane | 1.6121 |

| Example No. | Compound | Physical constant $n_D^{20}$ or mp. |
|---|---|---|
| 17 | 3-(3,4-dimethoxyphenyl)-3-[3-(3-phenoxyphenyl)propyl]thietane | 1.6046 |
| 18 | 3-(4-ethoxyphenyl)-3-[3-(4-fluoro-3-phenoxyphenyl)propyl]thietane | 1.5901 |
| 19 | 3-(4-methoxyphenyl)-3-[3-(3-phenoxyphenyl)-propyl]thietane | 1.6055 |
| 20 | 3-(4-chlorophenyl)-3-[3-(4-fluoro-3-phenoxyphenyl)propyl]thietane | 1.6053 |
| 21 | 3-(4-fluorophenyl)-3-[3-(3-phenoxyphenyl)-propyl]oxetane | 1.5761 |
| 22 | 3-(4-methylphenyl)-3-[3-(3-phenoxyphenyl)-propyl]oxetane | 1.5834 |
| 23 | 3-[3-(3-phenoxyphenyl)propyl]-3-(4-trifluoromethylphenyl)oxetane | 1.5503 |
| 24 | 3-[4-(2-fluoroethoxy)phenyl)-3-[3-(3-phenoxyphenyl)propyl]oxetane | 1.5767 |
| 25 | 3-[3-(3-phenoxyphenyl)propyl]-3-(4-propoxyphenyl)oxetane | 1.5748 |
| 26 | 3-[4-(1-methylethoxy)phenyl]-3-[3-(3-phenoxyphenyl)propyl]oxetane | 1.5715 |

| Example No. | Compound | Physical constant $n_D^{20}$ or mp. |
|---|---|---|
| 27 | 3-(3,4-methylenedioxy)phenyl)-3-[3-(3-phenoxyphenyl)propyl]oxetane | 1.5923 |
| 28 | 3-(4-ethoxy-3-fluoro)phenyl)-3-[3-(3-phenoxyphenyl)propyl]oxetane | 1.5713 |

The following Examples illustrate the possible uses of the compounds of the invention, that are in the form of the previosly mentioned preparations.

Example 29

Activity against larvae (L2) of the cotton army worm (*Spodoptera littoralis*)

Compounds of the invention were made up as aqueous emulsions at a concentration of 0.1%. Leaflet pairs of beans (*Vicia fabae*) as well as 10 larvae (L2) of the cotton army worm (*Spodoptera littoralis*) per experiment were sprayed with 4mg spray/$cm^2$ of these preparations in polystyrene petri dishes. The closed petri dishes were left in the laboratory under extended daylight conditions for two days. The % mortality of the larvae after two days indicated the level of activity.

In these experiments the compounds of Examples 1-28 caused 100% mortality.

Example 30

Activity against larvae (L3) of the Mexican bean beetle (Epilachna varivestis)

Compounds were made up as aqueous emulsions at a concentration of 0.1%. French bean plants (Phaseolus vulgaris) in the primary leaf stage were dipped in the preparations. For each test two plant stems with 4 primary leaves were placed in glass vases filled with water and enclosed in plexiglass cylinders. Then 5 larvae of the Mexican bean beetle (Epilachna varivestis) at the third larval stage were put in the glass cylinders and kept for 3 days. The % mortality of the larvae after 3 days indicated the level of activity.

In these experiments the compounds of Examples 1-28 caused 100% mortality.

Example 31

Insecticidal and acaricidal activity against Boophilus microplus (1), Lucilia sericata (2) Musca domestica (3) and Blattella germanica (4).

1. 9 cm diameter filter papers were impregnated with 1 ml aliquots of acetone solutions of test compound at various concentrations. The papers were allowed to dry and then folded into envelopes in which cattle tick larvae, (Boophilus microplus) were enclosed and held at 25°C and 80% R.H. for 48 hours. The

 

percentage mortality of tick larvae was then recorded and compared with controls.
The controls gave a mortality of less than 5% whereas compounds of Examples 1-7, 9-18 and 21-28 caused at least 50% mortality at a concentration of 300 ppm or less.

2. 1ml aliquots of an acetone solution containing test compound at various concentrations were applied to cotton wool dental rolls 1cm x 2cm, contained in glass vials (2cm diameter x 5cm long). After drying, the treated materials were then impregnated with 1ml of nutrient solution, infested with first instar larvae of sheep blowfly (<u>Lucilia</u> <u>sericata</u>), closed by a cotton wool plug and held at 25°C for 24 hours.
For the controls the mortality was <5% whereas the compounds of Examples 1-13, 15, 17, 18 and 21-28 had an $LC_{50}$ of 300 ppm or less.

3. Aliquots of acetone solutions of test compounds at various concentrations were applied to 9cm diameter filter papers placed in the bottom of 9cm diameter petri dishes closed by glass lids. After evaporation of solvent, the treated surfaces, together with control treated with acetone alone, were then infested with adult houseflies, (<u>Musca</u> <u>domestica</u>) and held at 22°C for 24 hours.

The percentage mortality of the insects was then recorded. Less than 5% mortality resulted in the control treatments whereas the compounds of Examples 1, 5, 9, 10 and 22-28 had an $LD_{50}$ of 1000 $mg/m^2$ or less.

4. Aliquots of acetone solutions of test compounds at various concentrations were applied to glass plates (10cm x 10cm). After evaporation of solvent, the treated surfaces, together with controls treated with acetone alone, were then infested with second instar nymphs of the German cockroach, (Blattella germanica), retained on the treated surface within PTFE-coated glass rings 6cm in diameter and held for 24 hours at 22°C. The percentage mortality of the insects was then recorded.
Less than 5% mortality resulted in the control treatments whereas the compounds of Examples 1-13, 15, 18, 19, 21-23, 25, 27 and 28 had an $LD_{50}$ of 300 $mg/m^2$ or less.

0984I

0216228

## CLAIMS

1. A compound of general formula I

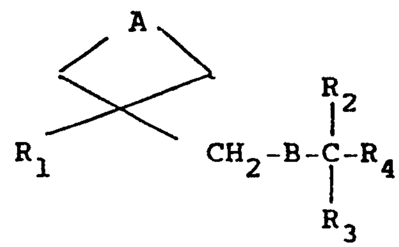

(I)

in which

$R_1$ is aryl or aryl substituted by $C_{1-4}$-alkyl, halo-$C_{1-4}$-alkyl, phenyl-$C_{1-4}$-alkyl, $C_{2-4}$-alkenyl, halo-$C_{2-4}$-alkenyl, phenyl-$C_{2-4}$-alkenyl, $C_{2-4}$-alkynyl, halo-$C_{2-4}$-alkynyl, phenyl-$C_{2-4}$-alkynyl, $C_{1-4}$-alkoxy, halo-$C_{1-4}$-alkoxy, phenyl-$C_{1-4}$-alkoxy, $C_{2-4}$-alkenyloxy, alkylsulphonyloxy, haloalkylsulphonyloxy, arylsulphonyloxy, halo-$C_{2-4}$-alkenyloxy, phenyl-$C_{2-4}$-alkenyloxy, halo, cyano, nitro, aryloxy, haloaryloxy, $C_{1-4}$-alkylaryloxy, or nitro-$C_{1-4}$-alkylaryloxy,

$R_2$ and $R_3$ are the same or different and are hydrogen, fluorine, cyano or ethynyl,

$R_4$ is phenyl or pyridyl or these groups substituted by one or more of $C_{1-6}$-alkyl, halo-$C_{1-6}$-alkyl, phenyl-$C_{1-6}$-alkyl,

$C_{1-6}$-alkyl interrupted by an O-, N- or S- atom, $C_{2-4}$-alkenyl, halo-$C_{2-4}$-alkenyl, phenyl-$C_{2-4}$-alkenyl, $C_{1-4}$-alkoxy, halo-$C_{1-4}$-alkoxy, phenyl-$C_{1-4}$-alkoxy, $C_{2-4}$-alkenyloxy, halo-$C_{2-4}$-alkenyloxy, phenyl-$C_{2-4}$-alkenyloxy, aryloxy, haloaryloxy, $C_{1-4}$-alkylaryloxy, arylamino, haloarylamino, $C_{1-4}$-alkylarylamino, aryl-N-$C_{1-4}$-alkylamino, aryl-N-$C_{1-4}$ acylamino, aroyl, haloaroyl, $C_{1-4}$-alkylaroyl, aryl, haloaryl, $C_{1-4}$-alkylaryl or halo,

A is O or S and

B is $CH_2$ or O.

2. A compound according to claim 1 in which $R_2$ and $R_3$ are both hydrogen and $R_4$ is 3-phenoxyphenyl or 4-fluoro-3-phenoxyphenyl.

3. A compound according to claim 1 or 2, in which A is O.

4. A compound according to claim 1, 2 or 3, in which B is $CH_2$.

5. A compound according to any one of the preceding claims, in which $R_1$ is phenyl, optionally substituted

in the 4-position or in both the 3- and 4- positions by halogen, $C_{1-4}$-alkyl, $C_{1-4}$-alkoxy, $C_{1-4}$-haloalkyl or $C_{1-4}$-haloalkoxy or two adjacent substituents form a methylenedioxy group.

6. A compound according to claim 5, in which $R_1$ is phenyl substituted in the 4-position by halogen, methyl, trifluoromethyl or ethoxy.

7. An insecticidal or acaricidal composition which comprises a compound according to any one of the preceding claims in admixture with conventional additives or carriers.

8. A process for the preparation of a compound of formula I according to claim 1 characterised by,

a) when A in formula I is oxygen, treating a compound of formula II

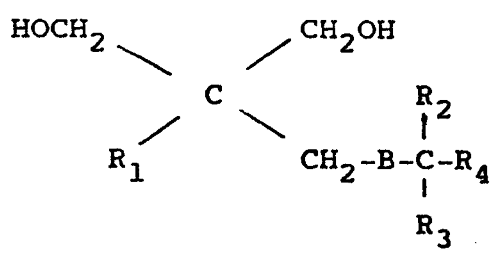

(II)

firstly with a sulphonyl chloride to give a compound of formula III

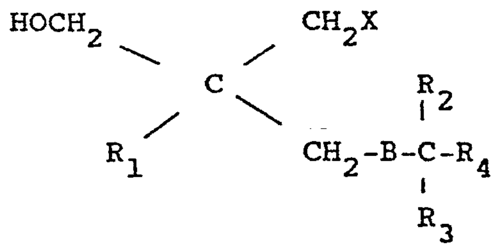 (III)

in which $R_1$, $R_2$, $R_3$, $R_4$ and B have the meanings given above and X is a leaving group, and then cyclising this to the oxetane, with a strong base, and b) when A in formula I is sulphur, treating a compound of formula II with a sulphonyl chloride to give an intermediate of general formula IV

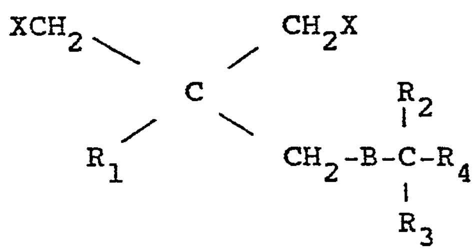 (IV)

in which $R_1$, $R_2$, $R_3$, $R_4$, X and B have the meanings given above and X is a leaving group, and then cyclising this to the thietane with sodium sulphide.

0984I

European Patent Office

# EUROPEAN SEARCH REPORT

0216228
Application number

EP 86112300.8

| Category | Citation of document with indication, where appropriate, of relevant passages | Relevant to claim | CLASSIFICATION OF THE APPLICATION (Int Cl 4) |
|---|---|---|---|
| A | DE - B2 - 1 793 773 (ESSO RESEARCH AND EGINEERING CO)<br>* Totality *<br>-- | 1,7,8 | C 07 D 305/06<br>C 07 D 331/04<br>C 07 D 407/04<br>C 07 D 409/04<br>C 07 D 409/08<br>C 07 D 409/12<br>A 01 N 43/20 |
| A | DE - A - 2 136 278 (COMMONWEALTH SCIENTIFIC AND INDUSTRIAL RESEARCH ORGANISATION)<br>* Claims 1,7,15 *<br>-- | 1,7,8 | |
| A | DE - A1 - 3 030 760 (RÜTGERS WERKE AG)<br>* Claim 1 *<br>-- | 1 | |
| D,A | EP - A1 - 0 104 908 (COMMON WEALTH SCIENTIFIC AND INDUSTRIAL RESEARCH ORGANISATION)<br>* Abstract *<br>---- | 1,7,8 | TECHNICAL FIELDS SEARCHED (Int Cl 4)<br>C 07 D 305/00<br>C 07 D 331/00<br>C 07 D 407/00<br>C 07 D 409/00<br>A 01 N 43/00 |

The present search report has been drawn up for all claims

| Place of search | Date of completion of the search | Examiner |
|---|---|---|
| VIENNA | 12-12-1986 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82